# EUROPEAN PATENT APPLICATION

(11) **EP 2 645 281 A2**
(43) Date of publication of application: **02.10.2013**
(21) Application number: 13161199.8
(22) Date of filing: 26.03.2013
(51) Int. Cl.: G06F 19/00

(54) **System and method of managing technician review of medical test data**

(30) Priority: 29.03.2012 US 201213433915
(71) Applicant: General Electric Company, Schenectady, New York 12345 (US)
(72) Inventor: Young, Brian J., Wauwatosa, WI 53213 (US)
(74) Representative: Illingworth-Law, William Illingworth

(57) **Abstract**

A system 10 and method 100 of managing a technician review of medical test data collected from a patient includes receiving medical test data 102. At least one analysis algorithm is applied to the medical test data to produce a pre-evaluation summary 104. The medical test data is assigned to the technician based upon an application of a triage rule to the pre-evaluation summary 106,108 and the assigned medical test data is presented in a queue of work for the technician 110.

## Description

The present disclosure is related to the field of medical test data analysis. More specifically, a system and method that schedules technician review of medical test data.

A Holter test generally refers to any of a variety of tests that continuously monitor electrical activity within the body over a period of time. These tests are often conducted over at least 24 hours; however, some Holter tests are conducted in as little as 12 hours or less, or can take as long as two or more weeks. Typically, the Holter test monitors the electrical activity of the cardiovascular system, but other biopotentials, exemplarily electroencephalography (EEG) can also be monitored. A Holter monitor is a portable device that uses a plurality of electrodes placed on the patient and continuously monitors the electrical activity in the patient over the testing period. Accordingly, a Holter test produces a large volume of electrical potential data that must be reviewed and analyzed before the test data can be used to inform diagnosis or treatment decisions by a care provider.

While computer-implemented algorithms can be used in systems to analyze the Holter test data to provide automated analyses, most medical care facilities employ technicians to manually review the Holter test data before any assessments or diagnoses are made. The reviewing technician uses analysis software as well as the technician's own experience and expertise to interpret and analyze the results. However, even with these tools, technician analysis of Holter test data can require anywhere between fifteen minutes and in excess of one hour of technician time for each test.

A method of managing a technician to review medical test data collected from a patient includes receiving medical test data. At least one analysis algorithm is applied to the medical test data to produce at least one pre-evaluation summary of the medical test data. At least one triage rule is applied to the at least one pre-evaluation summary of the medical test data. The medical test data is assigned to the technician and an indication of the medical test data is presented in a queue of work for the technician on a graphical display.

In one embodiment, a method of managing the assignment of medical test data for manual review by a technician among a plurality of technicians includes receiving medical test data of a completed medical test comprising a continuous physiological data recording. A medical test pre-evaluation is conducted that includes applying at least one clinician importance algorithm to the continuous physiological data recording to identify at least one clinical abnormality present in the continuous physiological data recording and applying at least one difficulty analysis algorithm to the continuous physiological data recording to estimate a signal quality of the continuous physiological data recording. An urgency of the medical test data is evaluated based upon the at least one identified clinical abnormality according to at least one triage rule. A processing time of the medical test data is evaluated based upon the estimated signal quality according to the at least one triage rule. A manual review of the medical test data is assigned to a technician of the plurality of technicians based upon the evaluated urgency and processing time of the medical test data. A work queue presented on a graphical display of the technician is updated to include an indication of the medical test data.

In one embodiment of a system for facilitating a review of medical test data by a technician includes a technician workstation. The technician workstation includes a graphical display that operates to present a work queue to the technician. The work queue includes indications of medical test data assigned to the technician for review. A first computer readable medium stores at least one clinical importance algorithm. A second computer readable medium stores at least one difficulty algorithm. A third computer readable medium stores at least one triage rule. A medical analysis computer receives medical test data. The medical test analysis computer creates a pre-evaluation of the medical test data by accessing and applying the at least one clinical importance algorithm and at least one difficulty algorithm to the medical test data. The medical analysis computer assigns the medical test data to the technician based upon the at least one triage rule and provides the indication of the medical test data to the technician workstation for presentation in the work queue.
Fig. 1 is a system diagram depicting one embodiment of a system for technician review of medical test data.
Fig. 2 is a flow chart that depicts one embodiment of a method of managing a technician review of medical test data.
Fig. 3 is a flow chart that depicts one alternative embodiment of a method of managing a technician review of medical test data.

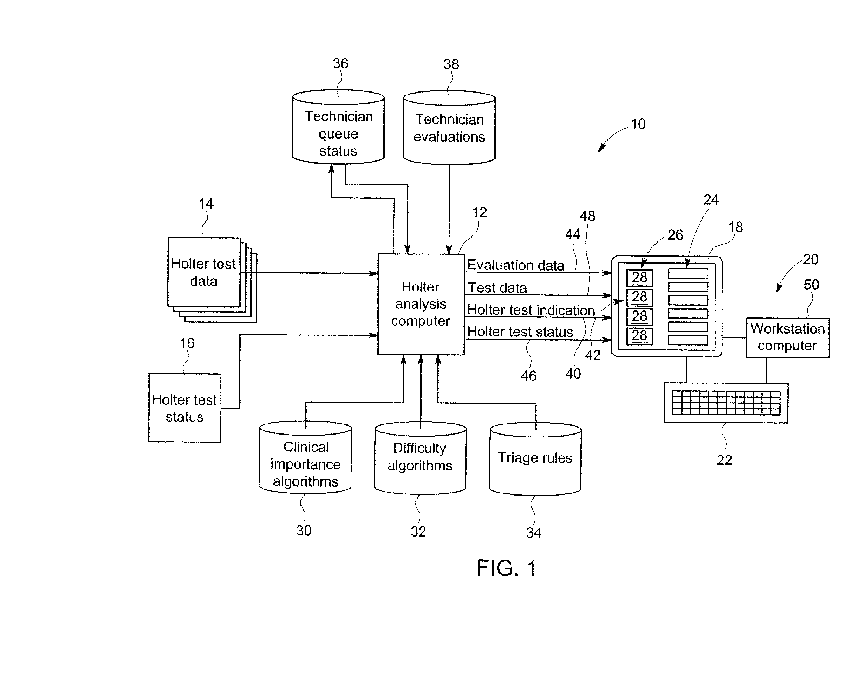

Fig. 1 is a system diagram that depicts one embodiment of a system for managing clinician review of medical test data 10. It is to be recognized that the specific medical test of a Holter test is exemplarily used throughout the application as one embodiment of the application of embodiments of the systems and methods as disclosed herein and this is in no way intended to be limiting on the scope of the medical test data that may be used in alternative embodiments. In a general sense, the medical test data is a continuously acquired physiological data recording. In alternative embodiments of the system 10, the medical test data can be non-invasively obtained blood pressure (NIBP), SpO₂, respiration or other physiological parameters. In still further embodiments, medical test data is a multiple parameter recording. Medical test data used in embodiments disclosed herein can further include continuous medical test data acquired from a stationary or ambulatory patient.

The system 10 uses a medical test analysis computer 12 (herein exemplarily referred to as Holter analysis computer 12) to receive and process data as disclosed herein. The Holter analysis computer 12 may be implemented as any of a variety of specific purpose or general purpose computers or computer processors that execute computer readable code stored upon computer readable media such as to carry out the functions as disclosed herein. As used herein, computer readable media refers to any of a variety of non-transient media including, but not limited to, volatile and non-volatile computer memory, e.g. flash memory. The Holter analysis computer 12 receives, accesses, processes, and/or transmits data and/or algorithms from a variety of sources as disclosed herein. Such sources may be remotely located from the Holter analysis computer 12, or alternatively, the sources of data and/or algorithms may be integrally connected to the Holter analysis computer or any other type of communicative connection as would be recognized by one of ordinary skill in the art.

The Holter analysis computer 12 receives medical test data 14 (herein exemplarily referred to as Holter test data 14) of a plurality of completed Holter tests. In a large medical care facility or system, the Holter analysis computer 12 can receive such Holter test data 14 for a completed Holter test in a variety of ways. These ways of receiving Holter test data include, but are not limited to, downloading from a server, such as an electronic medical record (EMR) server (not depicted) within which the Holter test data for the completed Holter test is stored. In alternative embodiments, the Holter monitor (not depicted) used to collect the Holter test data 14 during the now completed Holter test, is communicatively connected to the Holter analysis computer 12 by a wired or a wireless connection, and the Holter test data 14 is downloaded to the Holter analysis computer 12 directly from the Holter monitor. In a still further exemplary embodiment, a memory device from the Holter monitor, such as non-volatile memory which may exemplarily be flash or SD memory can be removed from the Holter monitor and read by the Holter analysis computer 12 in order to download the Holter test data 14 into the Holter analysis computer 12. While this disclosure has included descriptions of various ways in which the Holter test data 14 can be transferred to the Holter analysis computer 12, or received by the Holter analysis computer 12, a person of ordinary skill in the art will recognize other suitable data transfer techniques that are considered to be within the scope of the present disclosure.

In embodiments, the Holter analysis computer 12 further receives an indication of Holter test status 16, which may be provided to the Holter analysis computer 12 from a server in a hospital or medical care facility information network (not depicted). The Holter test status 16 includes an identification of scheduled, on-going, and completed Holter tests. As will be described in further detail herein, such an indication of Holter test status 16 provides information as to current and future Holter test data analysis workload in the system 10. In still further embodiments, the Holter test status can include any indications of expedited or "stat" orders placed on particular Holter tests by a clinician.

The Holter analysis computer 12 is further communicatively connected to a graphical display 18. The graphical display 18 may be a part of a Holter analysis workstation 20 that may further include a workstation computer 50 and an input device 22, such as a keyboard. The technician uses the workstation 20 to manage the technician's workload as well as to perform the manual review of the Holter test data assigned to the technician as will be described in further detail herein. In embodiments, the graphical display 18 can provide a presentation 24 of all of the Holter test statuses received by the Holter analysis computer 12, although not necessarily assigned to the technician. Additionally, the display 18 presents a work queue 26, which includes indications 28 of the Holter test data assigned to the technician for review and analysis.

In embodiments, the Holter analysis computer 12 can be connected to a variety of computer readable media (examples of which are disclosed above). The computer readable media store algorithms or supplemental data used by the Holter analysis computer 12 as described in further detail herein. Some of such computer readable media store algorithms accessed and used by the Holter analysis computer 12, such as clinician importance algorithms 30, difficulty algorithms 32, and triage rules 34. Other computer readable media store supplemental data that is used by the Holter analysis computer 12 in some embodiments. Particularly, the supplemental data is related to or evaluates the individual technicians of a plurality of technicians that review and analysis Holter test data within a medical care facility. Such computer readable media include those storing technician queue status 36 and technician evaluations 38. Each of these will be disclosed in further detail herein, particularly in the context of embodiments of methods that may operate within embodiments of the system 10.

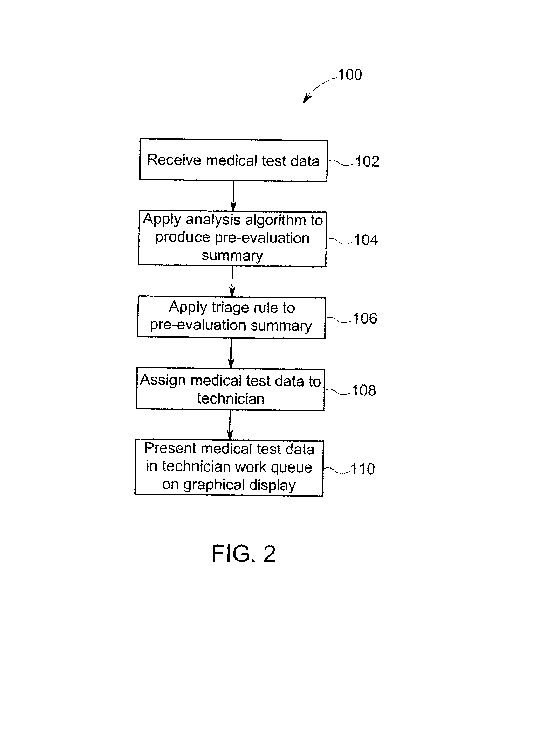

Fig. 2 is a flow chart that depicts one embodiment of a method 100 of managing a technician review of medical test data. At 102, the medical test data is received. The medical test data is described above and is exemplarily Holter test data that are one or more recordings of biopotential signals that exemplarily include cardiac biopotentials. The medical test data is continuously obtained rather than event-triggered and can be 24 hours or less of data or up to or exceed two weeks of continuously obtained biopotentials. The biopotential signals in the medical test data include physiological characteristics of clinical importance and also include noise from the collection process that can obscure the physiologically relevant features. Technicians are specifically trained to analyze and interpret the medical test data and to use signal analysis software to perform such analyses despite noise that may be presented in the data; however, levels and types of noise or types of clinically important information in the medical test data can affect the length of time that a technician requires in order to completely analyze and interpret the medical test data.

At 104 the Holter analysis computer applies at least one analysis algorithm to the received medical test data in order to produce a pre-evaluation summary. The at least one analysis algorithm can include a clinical importance algorithm such as from computer readable medium 30 and/or a difficulty algorithm from computer readable medium 32, as depicted in the system 10 of Fig. 1. Clinical importance algorithms can include similar features to any of a variety of automated data analysis or signal morphology detection algorithm as known. However, the clinical importance of algorithms are specifically tailored to identifying and quantifying the existence of clinical abnormalities in the medical test data for future analysis. In the context of Holter test data, such clinical abnormalities include, but are not limited to, ventricular tachycardia (VT), super ventricular tachycardia (SVT), couplets or runs of VT or SVT, R-wave pauses, torsades (TDP), or morphological indicators of sudden cardiac death (SCD). In embodiments, the application of at least one clinical importance algorithm produces a listing of the morphological features identified above found to be present in the Holter test data, the listing of which is reported in the pre-evaluation summary. In a still further embodiment, the pre-evaluation summary further includes a numerical count of occurrences of these morphological features, or a severity rating of the prevalence of the identified physiologically relevant features in the Holter test data. In embodiments, the clinical importance of algorithms define one or more of the identified clinical abnormalities in terms of a signal pattern or relationship between features in the Holter test data signals. As non-limiting examples, the clinical importance algorithms can define signal shapes or morphologies that are equated with one or more of the clinical abnormalities, or define a relationship between identified morphological features in the Holter test data, exemplarily intervals such as ST intervals, QT intervals, or R-R intervals.

The analysis algorithm applied at 104 to produce the pre-evaluation summary may further include a difficulty algorithm that provides an evaluation of the relative difficulty that a technician will have in manually reviewing the Holter test data, such that this evaluation can be included in the pre-evaluation summary. In general, the quality of the signals in the Holter test data generally determine the difficulty that a technician will have in analyzing and interpreting the Holter test data. Therefore, the difficulty algorithms in general measure the quality of the signals in the Holter test data.

In one example, the difficulty algorithm is applied to measure a signal to noise ratio of signal(s) in the Holter test data. This signal to noise ratio can be reported for either the entire signal as a whole, or alternatively, the signal in the Holter test data can be divided into temporal segments and a signal to noise ratio for each segment is evaluated. In a non-limiting example, the Holter test data can be divided into one hour segments, however, this is not limiting on the time length of the segments as any time length could be selected for such intervals, and a signal to noise ratio calculated for each of the segments. In a still further embodiment, the pre-evaluation summary reports the signal to noise ratio for each of the segments as a count or a percentage of the overall Holter test data that meets certain signal to noise ratio thresholds. Therefore, the pre-evaluation summary may include a report that exemplarily 20% of the Holter test data is high quality, 50% of the Holter test data is average quality, and 30% of the Holter test data is poor quality as compared to signal to noise ratio threshold values.

In an alternative embodiment, the difficulty algorithm incorporates pattern matching, the pattern matching comprises a plurality of patterns or templates that are representative of various forms of signal quality or patterns of noise. In such embodiments, the pattern matching difficulty algorithm identifies the number of different noise or signal quality patterns that are present in the Holter test data and provides a report of the number and frequency of the patterns found. The number and frequency of such patterns identified in the Holter test data is correlated to the quality of the signals in the Holter test data. In general terms, the more different noise patterns found in the test data and the greater frequency with which these patterns are matched, the overall lower quality of the Holter test data and the more difficult the Holter test data will be for the technician to analyze and interpret.

At 106 a triage rule, such as from the computer readable medium 34 storing triage rules in the system 10 of Fig. 1, is applied to the pre-evaluation summary that may include an analysis of the clinical importance of the Holter test data, and/or an analysis of the general difficulty that the Holter test data will present to a reviewing technician. The application of the triage rules at 106 assist at 108 to assign the medical test data to a technician for review and interpretation.

In embodiments, the triage rules can be defined by a medical care institution, or can be acquired from alternative sources, including default general triage rules from a provider of a commercial embodiment of the system or method. The triage rules are applied at 106 to the information in the pre-evaluation summary in order to evaluate an urgency attributed to the clinical abnormalities identified by the clinical importance algorithms and to assign the analysis of Holter test data between technicians based upon the urgency or difficulty of the Holter test data. More skilled or experienced technicians generally are able to complete analysis and interpretation of Holter test data that is more difficult or contains more complex clinical abnormalities; however, the greater clinical importance or urgency of the features identified in the Holter test data is relevant to select a technician that is available to begin the analysis and interpretation quickly. Therefore, in an embodiment as disclosed in more detail herein, the triage rules may further evaluate a current workload of the technician before the Holter test data is assigned to the technician at 108.

After the Holter test data is assigned to a technician at 108, the Holter test data is presented to the assigned technician in a work queue on a graphical display associated with that technician. As will be described in more detail herein, in medical care facilities that include a plurality of technicians to review medical test data, management of a work queue of medical test data for the technician provides the technician with an up-to-date listing of the medical test data assigned to that technician for review, and presents the medical test data in an optimized order based upon the triage rules as discussed above and as further discussed herein to account for difficulty, urgency, and/or age of the medical test data to be reviewed.

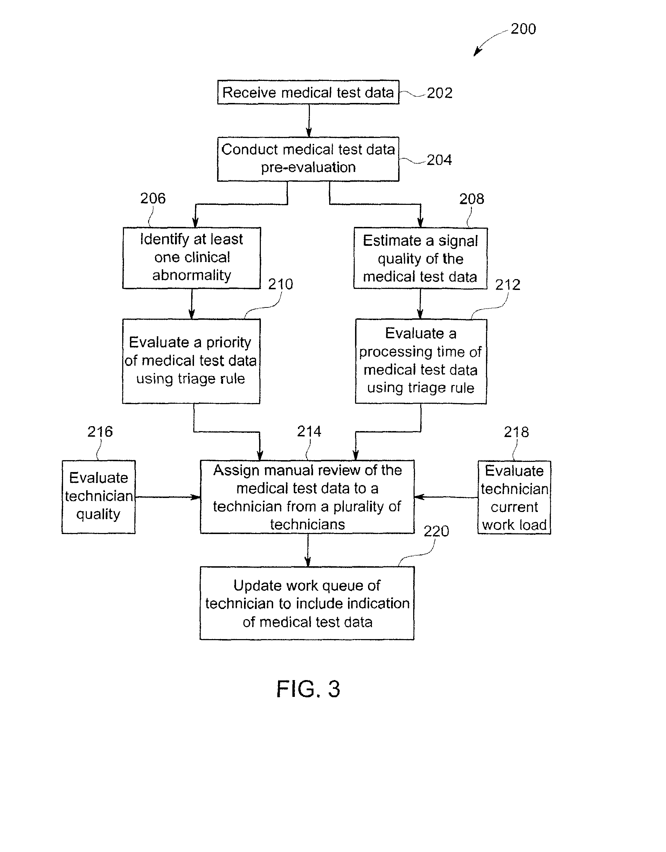

Fig. 3 is a flow chart that depicts one embodiment of a method 200 of managing the assignment of medical test data for manual review by a technician among a plurality of technicians. It will be recognized that some embodiments of the method 200 include the general features of the method 100, and to such extent, present more detailed versions thereof.

Medical test data, exemplarily Holter test data, is received by the Holter analysis computer at 202. Next, at 204, a medical test data pre-evaluation is conducted. Similar to embodiments disclosed above, the pre-evaluation of 204 uses a clinical importance algorithm and a difficulty algorithm to identify at least one clinical abnormality at 206 and estimate a signal quality of the medical test data at 208, respectively. It is to be understood that some embodiments may use one or more clinical importance algorithm or difficulty algorithm and apply those to the received medical test data in arriving at the identified at least one clinical abnormality 206 and estimate of a signal quality of the medical test data at 208.

At 210 the identified at least one clinical abnormality from 206 is used to evaluate an urgency of the medical test data using at least one triage rule. As disclosed above, the identified at least one clinical abnormality can either be in the pre-evaluation in the form of a list of one or more identified clinical abnormalities found in the medical test data, or can include a numerical count of the occurrences of each identified type of clinical abnormality. In a still further embodiment, an evaluation or rating of identified clinical abnormalities is provided. Thus, the identification of the at least one clinical abnormality from 206 is used at 210 in evaluating the urgency of the medical test data according to at least one triage rule. The at least one triage rule identifies a priority level for the technician analysis of the medical test data based upon the identified at least one clinical abnormality. In a still further embodiment, the clinical test data may further include a "stat" order provided by a clinician, which may be interpreted by a triage rule to move the technician review of this particular medical test data to the highest priority. Alternatively, the triage rule may identify medical test data that exhibits the most severe or life threatening clinical abnormality to be of a high priority, such that the analysis and interpretation are provided back to an attending clinician in a shorter time period compared to medical test data that exhibits a relatively normal physiological condition or function.

At 212 the estimate of signal quality of the medical test data from 208 is used to evaluate a processing time of the medical test data by using at least one triage rule. In addition to the evaluation of urgency or priority of medical test data, the triage rules can further define how the relative difficulty presented by signal quality in medical test data should be considered or handled in the context of assigning a technician for analysis and interpretation of the medical test data. As disclosed above, generally, a lower quality signal, exhibiting more noise or signal artifacts, requires a greater amount of processing time for the technician to analyze and interpret the medical test data. The implications of specific degradations of the signal quality as identified at 208 can be defined in the triage rules by the medical care institution such as to note specific types of noise or signal degradation present particular difficulty to technicians in analyzing and interpreting medical test data.

The evaluations of a priority of the medical test data from 210 and a processing time of the medical test data from 212 are used at 214 to assign the manual review of the medical test data to a technician from a plurality of technicians. The assignment of the medical test data to a specific technician at 214 can be based upon the priority of the medical test data and the required processing time of the medical test data. To further this process by applying the triage rules, an evaluation of technician quality can be provided at 216 and an evaluation of a technician's current workload can be provided at 218. As noted above, in general, a higher quality or more experienced technician will generally more accurately and more quickly analyze and interpret medical test data. Therefore, in embodiments, triage rules can exhibit a preference to assign higher priority or more complex medical test data to technicians that have been evaluated to be of higher quality or proficiency or deemed to have particular expertise in analysis and interpretation of clinical abnormalities identified to be in the medical test data. This can promote time efficiency among technician assignments as higher quality, more proficient, or more experienced technicians can analyze and interpret medical test data more quickly.

Additionally, by evaluating the current workloads of each of the plurality of technicians, as provided at 218, the workloads can be balanced across technicians taking into account the relative difficulty or complexity of each of the medical tests currently assigned to each of the technicians and the clinician's relative ability to analyze and interpret the medical test data already assigned. Estimates of processing time from 212 can be used to provide an estimate of the total amount of work currently assigned to each technician. In still further embodiments, the evaluations of the priority of the medical test data is first compared across all of the medical test data queued for technician review to determine a test's priority in view of currently assigned tests. Then each technician's workload and the estimated processing times are used to assign the medical test data to a technician such that the medical test data will be assigned to a technician such that overall, the medical test data will be processed in the order as determined by the determined priority.

The evaluation of technician quality provided at 216 can come from a variety of sources. Such evaluations can be routinely made as a part of employment of technicians and be based upon interviews or other such work evaluations conducted by a manager. Alternatively, the evaluation can come from manual or automated review or evaluation of the work performed by the technician over a time period. In still further embodiments, the evaluation of technician quality can be obtained from a periodic test administered to technicians such as to provide a periodic evaluation of technician quality. Such a periodic test can be automated and occur on a schedule.

Once the medical test is assigned to a technician from the plurality of technicians at 214, the work queue of the selected technician is updated at 220 to include an indication of the medical test data. In addition to using the priority of the medical test data and the processing time of the medical test data as determined at 210 and 212 to assign the medical test data to a technician at 214, these evaluations can also be used in order to locate the medical test data within the work queue of the assigned technician. Therefore, if medical test data is evaluated to be of a high priority, then the indication of the medical test data is placed at or near the top of the technician's work queue, indicating the priority of that medical test data over other medical test date that has been previously assigned to that technician. In still further embodiments, the work queue is also weighted based upon time since the Holter test data was collected, therefore even low priority test data is analyzed by a technician in a timely manner.

Returning to the system 10 depicted in Fig. 1, the Holter analysis computer 12, after executing computer readable code stored on non-transient computer readable media to carry out one embodiment of the method as described above, provides the Holter test indication 40 to the graphical display 18 for presentation in the work queue 26 on the graphical display 18. The new indication of assigned Holter test data 42 is added into the work queue 26 at a position as defined based upon the previously discussed evaluations of Holter test data priority and processing time.

In addition, the Holter analysis computer 12 can provide evaluation data 44 such as the evaluated priority of the medical test data or the evaluated processing time of the medical test data to the graphical display 18 for presentation along with the Holter test indications 28. As previously disclosed, the Holter analysis computer 12 can provide the received Holter test status 46 to the graphical display such that all of the statuses of ongoing Holter tests can be presented at 24. In still further embodiments, the Holter test data 48 is provided from the Holter analysis computer 12 to the graphical display 18, and workstation 20, such that the technician working at the workstation 20 can select the Holter test indication 28 with the user input device 22 and the graphical display 18 will present the Holter test data 48, along with any test data analysis software or tools that are available to the technician in analyzing and interpreting the Holter test data 48. Such software or tools can be stored at the Holter analysis computer 12, or at the workstation computer 50, or at another accessible computer readable media (not depicted).

This written description uses examples to disclose the invention, including the best mode, and also to enable any person skilled in the art to make and use the invention. The patentable scope of the invention is defined by the claims, and may include other examples that occur to those skilled in the art. Such other examples are intended to be within the scope of the claims if they have structural elements that do not differ from the literal language of the claims, or if they include equivalent structural elements with insubstantial differences from the literal languages of the claims.

Various aspects and embodiments of the present invention are defined by the following numbered clauses:
1. A method of managing of a technician to review medical test data collected from a patient, the method comprising:
   receiving medical test data;
   applying at least one analysis algorithm to the medical test data to produce at least one pre-evaluation summary of the medical test data;
   applying at least one triage rule to the at least one pre-evaluation summary of the medical test data;
   assigning the medical test data to the technician based upon the application of the at least one triage rule to the at least one pre-evaluation summary;
   presenting an indication of the medical test data in a queue of work for the technician on a graphical display.
2. The method of clause 1, wherein applying at least one analysis algorithm comprises:
   applying at least one clinical importance algorithm to the medical test data;
   identifying a physiological characteristic in the medical test data from the application of the at least one clinical importance algorithm to the medical test data;
   applying at least one difficulty analysis algorithm to the medical test data; and
   producing an evaluation of a signal quality of the medical test data from the application of the at least one difficulty analysis algorithm to the medical test data;
   wherein the pre-evaluation summary comprises the physiological characteristic and the evaluation of the signal quality.
3. The method of clause 1 or clause 2, wherein the technician is one of a plurality of technicians and further comprising:
   determining an ability of each technician of the plurality of technicians; and
   determining an availability of each technician at least one technician;
   wherein the application of the at least one triage rule uses the determined ability and the determined availability to assign the medical test data to a technician of the plurality of technicians.
4. The method of any preceding clause, further comprising:
   reordering the queue of work for the technician with the assigned medical test data based upon the physiological characteristic and the evaluation of signal quality; and
   presenting the pre-evaluation summary on the graphical display with the queue of work; and
   receiving a selection from the technician of medical test data for manual review from the queue of work presented on the graphical display in the order presented in the queue.
5. The method of any preceding clause, further comprising:
   weighting the identified physiological characteristic and evaluation of the signal quality of the pre-evaluation summary according to the at least one triage rule;
   wherein the indication of the medical test data is located in the queue of work for the technician based upon the identified physiological characteristic and evaluation of the signal quality weighted according to the at least one triage rule.
6. The method of any preceding clause, wherein the at least one clinical importance algorithm, when applied to the medical test data, identifies clinical abnormalities present in the medical test data and the physiological characteristic is a list of the identified clinical abnormalities.
7. The method of any preceding clause, wherein the list of the identified clinical abnormalities includes an indication of the prevalence of the clinical abnormalities in the medical test data.
8. The method of any preceding clause, wherein the difficulty analysis algorithm applies a plurality of waveform templates, and the evaluation of the signal quality is a count of the number of templates matched by at least a portion of the medical test data.
9. The method of any preceding clause, wherein the evaluation of the signal quality from the difficulty analysis algorithm is a signal to noise ratio for the medical test data.
10. The method of any preceding clause, wherein the medical test data is Holter test data.
11. A method of managing the assignment of medical test data for manual review by a technician among a plurality of technicians, the method comprising:
   receiving medical test data of a completed medical test comprising a continuous physiological data recording;
   conducting a medical test data pre-evaluation comprising:
      applying at least one clinical importance algorithm to the continuous physiological data recording test data to identify at least one clinical abnormality present in the continuous physiological data recording; and
      applying at least one difficulty analysis algorithm to the continuous physiological data recording to estimate a signal quality of the continuous physiological data recording;
      evaluating an urgency of the medical test data based upon the at least one identified clinical abnormality according to at least one triage rule;
      evaluating a processing time of the medical test data based upon the estimated signal quality according to the at least one triage rule;
      assigning the manual review of the medical test data to a technician of the plurality of technicians based upon the evaluated urgency and processing time of the continuous physiological data recording; and
      updating a work queue presented on a graphical display of the technician to include an indication of the medical test data.
12. The method of any preceding clause, further comprising:
   evaluating a work queue of each technician of the plurality of technicians based upon indications of medical test data present in the work queue to determine a time availability of each technician of the plurality of technicians;
   wherein the manual review of the medical test data is assigned to the technician of the plurality of technicians based upon the determined time availability of the technician.
13. The method of any preceding clause, further comprising:
   evaluating each of the technicians of the plurality of technicians on the basis of a quality of manual review performed by each of the technicians;
   wherein the manual review of the medical test data is assigned to the technician of the plurality of technicians further based upon the evaluated quality of each of the plurality of technicians.
14. The method of any preceding clause, wherein updating the work queue presented on the graphical display further comprises locating the indication of the medical test data within the work queue based upon the evaluated urgency and processing time of the medical test data.
15. The method of any preceding clause, wherein the identification of at least one clinical abnormality present in the continuous physiological data recording further comprises providing a count of each occurrence of the identified at least one clinical abnormality present in the continuous physiological data recording.
16. The method of any preceding clause, wherein the at least one difficulty analysis algorithm includes a plurality of signal templates and the estimation of the signal quality of the continuous physiological data recording comprises a count of the signal templates of the plurality matched by portions of the continuous physiological data recording.
17. A system for facilitating the review of medical test data by a technician, the system comprises:
   a technician workstation comprising a graphical display, the graphical display operates to present a work queue to the technician, the work queue including indications of medical test data assigned to the technician for review;
   a first computer readable medium that stores at least one clinical important algorithm that when applied to medical test data, identifies at least one clinical abnormality in the medical test data;
   a second computer readable medium that stores at least one difficulty algorithm that when applied to medical test data, evaluates a signal quality of the medical test data;
   a third computer readable medium that stores at least one triage rule; and
   a medical test analysis computer that receives medical test data, creates a pre-evaluation of the medical test data by accessing and applying the at least one clinical importance algorithm and at least one difficulty algorithm to the medical test data, assigns the medical test data to the technician based upon the at least one triage rule, and provides indication of the medical test data to the technician workstation for presentation in the work queue.
18. The system of any preceding clause, wherein the technician is one technician of a plurality of technicians, and the technician is selected based upon the application of the at least one triage rule to the medical test data by the medical test analysis computer.
19. The system of any preceding clause, further comprising a fourth computer readable medium that stores a queue status of each technician of the plurality of technicians and the technician of the plurality of technicians is selected by the medical test analysis computer based upon at least in part, the queue status of each technician and the at least one triage rule.
20. The system of any preceding clause, further comprising a fourth computer readable medium that stores a technician evaluation of each technician of the plurality of technicians and the technician of the plurality of technicians is selected by the medical test analysis computer based upon at least in part, the technician evaluation of each technician and the at least one triage rule.

## Claims

1. A method (100) of managing of a technician to review medical test data collected from a patient, the method comprising:
receiving medical test data (102);
applying at least one analysis algorithm to the medical test data to produce at least one pre-evaluation summary of the medical test data (104);
applying at least one triage rule to the at least one pre-evaluation summary of the medical test data (106);
assigning the medical test data to the technician based upon the application of the at least one triage rule to the at least one pre-evaluation summary (108);
presenting an indication of the medical test data in a queue of work for the technician on a graphical display (110).

2. The method of claim 1, wherein applying at least one analysis algorithm comprises:
applying at least one clinical importance algorithm to the medical test data;
identifying a physiological characteristic in the medical test data from the application of the at least one clinical importance algorithm to the medical test data;
applying at least one difficulty analysis algorithm to the medical test data; and
producing an evaluation of a signal quality of the medical test data from the application of the at least one difficulty analysis algorithm to the medical test data;
wherein the pre-evaluation summary comprises the physiological characteristic and the evaluation of the signal quality.

3. The method of claim 1 or claim 2, wherein the technician is one of a plurality of technicians and further comprising:
determining an ability of each technician of the plurality of technicians; and
determining an availability of each technician at least one technician;
wherein the application of the at least one triage rule uses the determined ability and the determined availability to assign the medical test data to a technician of the plurality of technicians.

4. The method of any preceding claim, further comprising:
reordering the queue of work for the technician with the assigned medical test data based upon the physiological characteristic and the evaluation of signal quality; and
presenting the pre-evaluation summary on the graphical display with the queue of work; and
receiving a selection from the technician of medical test data for manual review from the queue of work presented on the graphical display in the order presented in the queue.

5. The method of any preceding claim, further comprising:
weighting the identified physiological characteristic and evaluation of the signal quality of the pre-evaluation summary according to the at least one triage rule;
wherein the indication of the medical test data is located in the queue of work for the technician based upon the identified physiological characteristic and evaluation of the signal quality weighted according to the at least one triage rule.

6. The method of any preceding claim, wherein the at least one clinical importance algorithm, when applied to the medical test data, identifies clinical abnormalities present in the medical test data and the physiological characteristic is a list of the identified clinical abnormalities.

7. The method of any preceding claim, wherein the list of the identified clinical abnormalities includes an indication of the prevalence of the clinical abnormalities in the medical test data.

8. The method of any preceding claim, wherein the difficulty analysis algorithm applies a plurality of waveform templates, and the evaluation of the signal quality is a count of the number of templates matched by at least a portion of the medical test data.

9. The method of any preceding claim, wherein the evaluation of the signal quality from the difficulty analysis algorithm is a signal to noise ratio for the medical test data.

10. The method of any preceding clause, wherein the medical test data is Holter test data.

11. A method of managing the assignment of medical test data for manual review by a technician among a plurality of technicians, the method comprising:
receiving medical test data of a completed medical test comprising a continuous physiological data recording;
conducting a medical test data pre-evaluation comprising:
applying at least one clinical importance algorithm to the continuous physiological data recording test data to identify at least one clinical abnormality present in the continuous physiological data recording; and
applying at least one difficulty analysis algorithm to the continuous physiological data recording to estimate a signal quality of the continuous physiological data recording;
evaluating an urgency of the medical test data based upon the at least one identified clinical abnormality according to at least one triage rule;
evaluating a processing time of the medical test data based upon the estimated signal quality according to the at least one triage rule;
assigning the manual review of the medical test data to a technician of the plurality of technicians based upon the evaluated urgency and processing time of the continuous physiological data recording; and
updating a work queue presented on a graphical display of the technician to include an indication of the medical test data.

12. A system for facilitating the review of medical test data by a technician, the system comprises:
a technician workstation comprising a graphical display, the graphical display operates to present a work queue to the technician, the work queue including indications of medical test data assigned to the technician for review;
a first computer readable medium that stores at least one clinical important algorithm that when applied to medical test data, identifies at least one clinical abnormality in the medical test data;
a second computer readable medium that stores at least one difficulty algorithm that when applied to medical test data, evaluates a signal quality of the medical test data;
a third computer readable medium that stores at least one triage rule; and
a medical test analysis computer that receives medical test data, creates a pre-evaluation of the medical test data by accessing and applying the at least one clinical importance algorithm and at least one difficulty algorithm to the medical test data, assigns the medical test data to the technician based upon the at least one triage rule, and provides indication of the medical test data to the technician workstation for presentation in the work queue.

13. The system of claim 12, wherein the technician is one technician of a plurality of technicians, and the technician is selected based upon the application of the at least one triage rule to the medical test data by the medical test analysis computer.

14. The system of claim 12 or claim 13, further comprising a fourth computer readable medium that stores a queue status of each technician of the plurality of technicians and the technician of the plurality of technicians is selected by the medical test analysis computer based upon at least in part, the queue status of each technician and the at least one triage rule.

15. The system of any of claims 12 to 14, further comprising a fourth computer readable medium that stores a technician evaluation of each technician of the plurality of technicians and the technician of the plurality of technicians is selected by the medical test analysis computer based upon at least in part, the technician evaluation of each technician and the at least one triage rule.
